# EUROPEAN PATENT SPECIFICATION

(11) **EP 1 852 415 B1**
(45) Date of publication and mention of the grant of the patent: **07.10.2009**
(21) Application number: 07015051.1
(22) Date of filing: 09.07.2003
(51) Int. Cl.: C07C 213/08

(54) **Process for the preparation of N-monosubstituted beta-amino alcohols**
Verfahren zur Herstellung von N-Monoalkyl-Beta-Aminoalkoholen
Procédé pour la préparation de N-monoalkyl-beta-amino alcools

(30) Priority: 09.07.2002 EP 02015229
(43) Date of publication of application: 07.11.2007
(62) Divisional of application: 03762669.4
(73) Proprietor: Lonza AG, 4052 Basel (CH)
(72) Inventor: Michel, Dominique, Dr., 3960 Sierre (CH)

(56) References cited:
- EP-A- 0 046 288
- EP-A- 0 457 559
- EP-A- 0 650 965
- F. F. BLICKE: "The Mannich reaction" ORGANIC REACTIONS, vol. I, 1942, pages 303-341, XP002218609
- W. LEWIS ET AL.: "Ketonic Mannich bases..." JOURNAL OF THE AMERICAN PHARMACEUTICAL ASSOCIATION, vol. 47, no. 2, 1958, pages 77-81, XP001115095

## Description

The invention relates to a process for the synthesis of *N*-monosubstituted β-keto amines of formula 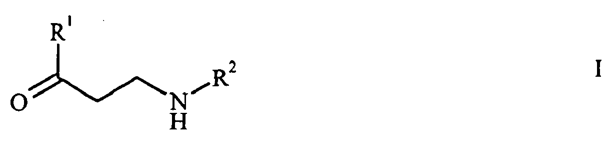 and/or an addition salt of a proton acid.

*N*-Monosubstituted β-keto amines of formula I are useful key intermediates for the preparation of pharmaceutically active compounds like (*S*)-duloxetine (Liu, H. et al., Chirality 12 (2000) 26-29), a potential neuro-active compound which strongly inhibits the serotonine and norephedrine uptake (Deeter, J. et al., Tetrahedron Lett. 31 (1990) 7101-7104).

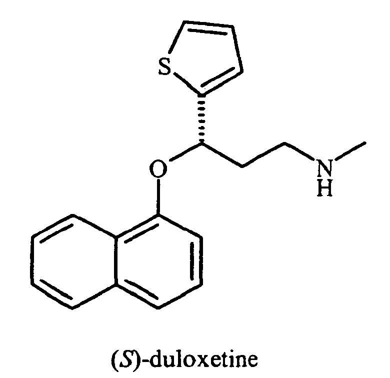

In the following the terms "amine" or "amines" include their corresponding addition salts of proton acids.

Direct preparation of *N*-monosubstituted β-keto amines of formula I establishes an alternative and economically advantageous source for industrial production of the corresponding *N*-monosubstituted β-amino alcohols.

Compounds of formula I were first synthesized in 1922 by reacting ketones with formaldehyde and primary or secondary alkylamines in the presence of hydrochloric acid ( Mannich, C. et al., Chem. Ber. 55 (1922) 356-365). In said reactions with primary alkylamines formation of hydrochlorides of tertiary β-keto amines of formula 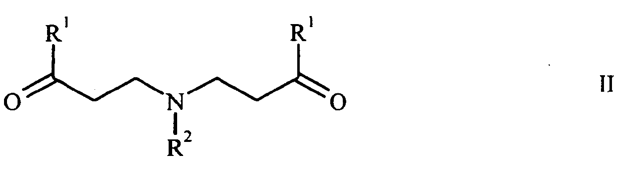 prevails over formation of hydrochlorides of secondary β-keto amines of formula I. These findings were supported by Blicke et al. (J. Am. Chem. Soc. 64 (1942) 451-454) and Becker et al. (Wiss. Z. Tech. Hochsch. Chem. Leuna-Merseburg. 11 (1969) 38-41).

According to Mannich et al. steam destillation of tertiary β-keto amines of formula II results in formation of secondary β-keto amines of formula I in fairly satisfactory yields, accompanied by vinyl compounds and other by-products.

In spite of the loss of more than 50 % of the starting compounds and due to lack of alternative processes this procedure is still used for the preparation of secondary β-keto amines.

Also from Blicke, F.F., Organic reactions, Vol. 1, Chapter 10, The Mannich Reaction, 1942, 303-341 is known that the Mannich reaction using primary amines leads to many side products.

Nobles, L.W. et al., J. Am. Pharm. Assoc., Sci. Ed., 67, 1958, 77-81 disclose among many compounds according to the compounds of formula II having a *N,N*-disubstituted amino group only one compound having a benzylamino group which is obtained in low yield. Another drawback in presently known preparation methods of β-keto amines is the need of isolation of the desired intermediate compounds of formula II from unwanted by-products of formula III.

EP-A 457 559 and EP-A 650 965 disclose the preparation of *N,N*-dimethyl β-amino alcohols via Mannich-type reactions of methyl ketones with paraformaldehyde and dimethylamine followed by reduction of the carbonyl group. After reaction of the hydroxyl group affording alkyl or aryl ether derivatives one methyl radical is removed to obtain *N*-monosubstituted compounds which requires delicate and expensive reactions.

Only Becker et al. disclose some few examples with yields of about 60% of *N*-monomethyl β-keto amines using *N*-methylammonium oxalates as nitrogen source. Nevertheless, the process disclosed by Becker et al. is not advantageous because it strictly depends on the use of amino oxalates. In contrast to the free amines or corresponding hydrochlorides oxalates of primary amines are not commercially available and their preparation requires further synthesis and purification steps.
Using oxalates is also disadvantageous because it requires additional reduction equivalents in the next step, reducing the ketone intermediates to the title compounds.

Although still many efforts were made to find new preparation processes, the pathway of the present invention for direct synthesis of *N*-monosubstituted β-keto amines as a possible source for subsequent reduction to *N*-monosubstituted β-amino alcohols is not yet disclosed.

The problem to be solved was to provide a process for the synthesis of *N*-monosubstituted β-keto amines and derivatives thereof. Furthermore, the proposed process should provide high yields independently of steric aspects of the used amino or carbonyl compounds.

The problems mentioned above could be solved according to claim 1.

Starting with commercially available methyl ketones and primary amines and/or an addition salt of a proton acid, which were reacted with formaldehyde in the presence a solvent and optionally of a proton acid at a pressure above 1.5 bar *N-*monosubstituted β-keto amines which could be directly reduced to the desired *N*-monosubstituted β-amino alcohols were obtained in high yields.

As a further advantage of the instant process high yields of *N*-monomethyl β-keto amines can be obtained by direct usage of methylamine hydrochloride which is easily available, cheap and, since it is a solid compound, easy to handle.

The present invention provides a process for the preparation of a compound of formula 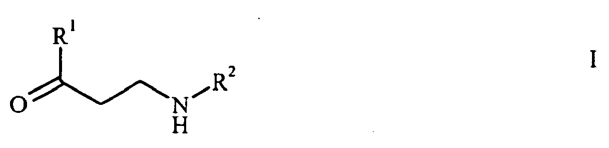 and/or an addition salt of a proton acid, wherein R¹ and R² independently represent alkyl, cycloalkyl, aryl or aralkyl, each being optionally further substituted with alkyl, alkoxy and/or halogen,
which process comprises reacting a mixture comprising
(i) a methyl ketone of formula 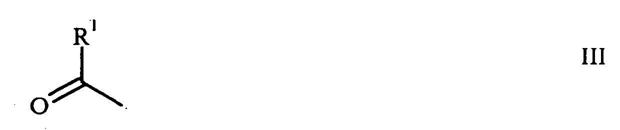 wherein R¹ is as defined above, and
(ii) a compound of formula

   H₂N-R² IV

   and/or an addition salt of a proton acid, wherein R² is as defined above, and
(iii) formaldehyde or a source of formaldehyde selected from the group consisting of formaldehyde in aqueous solution, 1,3,5-trioxane, paraformaldehyde and mixtures thereof, in the presence of
   a solvent selected from the group consisting of water, aliphatic alcohols, cycloaliphatic alcohols and mixtures thereof, and
   optionally a proton acid
to afford a compound of formula 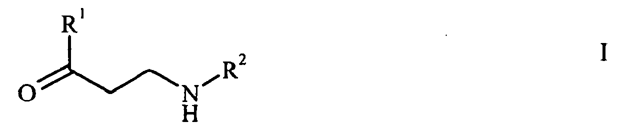 and/or an addition salt of a proton acid, wherein R¹ and R² are as defined above, and wherein the reaction is carried out at a pressure above 1.5 bar.

In a preferred embodiment R¹ and R² independently represent
linear or branched C₁₋₈ alkyl, C₃₋₈ cycloalkyl, phenyl, naphthyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅.

It is particularly preferred that R¹ represents furanyl or thienyl. It is also particularly preferred that R² represents linear or branched C₁₋₈ alkyl. More particularly preferred R² represents methyl, ethyl, propyl, isopropyl, butyl, isobutyl or tert-butyl.

Preferably, the compound of formula V can be used as a free amine and/or an addition salt of a proton acid thereof. Particularly preferred are free amines, formates, acetates, oxalates, hydrochlorides, hydrobromides or mixtures thereof. More particularly preferred are free amines and/or hydrochlorides.

In one preferred embodiment the compound of formula IV is present in an amount at least equimolar to that of the compound of formula III. Particularly preferred the molar ratio of the compound of formula IV to the compound of formula III is between 1 and 2.

In a preferred embodiment the solvent comprises water, an aliphatic or cycloaliphatic alcohol or a mixture thereof.

Particularly preferred alcohols are linear or branched aliphatic C₁₋₁₂ alcohols, cycloaliphatic C₅₋₈ alcohols, di- and/or trimeric ethylene glycols or mono C₁₋₄ alkyl or acetyl derivatives thereof, each of said alcohols containing 1 to 3 hydroxy groups.

Examples for said alcohols are methanol, ethanol, propanol, isopropyl alcohol, butanol, isobutanol, *tert*-butanol, 2-butanol, 1-pentanol, 2-pentanol, 3-pentanol, 1-hexanol, 2-hexanol, cyclopentanol, cyclohexanol, 1,2-ethanediol, 1,2-propanediol, 1,2-butanediol, 2,3-butanediol, 1,4-butanediol, 1,2,3-propanetriol, 1,2,6-hexanetriol, diethylene glycol, diethylene glycol monomethyl ether, diethylene glycol monoethyl ether, diethylene glycol monobutyl ether, diethylene glycol monoacetate, triethylene glycol, triethylene glycol monomethyl ether, triethylene glycol monoethyl ether, triethylene glycol monobutyl ether and triethylene glycol monoacetate.

Preferably said alcohol is ethanol, propanol, isopropyl alcohol, butanol, isobutanol, *tert*-butanol, diethylene glycol or triethylene glycol.

The proton acid can be any organic or inorganic acid, the acid being preferably selected from the group consisting of formic acid, acetic acid, propionic acid, oxalic acid, malonic acid, benzoic acid, HF, HCl, HBr, HI, H₂SO₄ and H₃PO₄. In a preferred embodiment the proton acid is an acidic salt of a polybasic organic or inorganic acids like monoalkali malonates, alkali hydrogensulfates, alkali hydrogenphosphates and alkali hydrogencarbonates. More preferably the proton acid is selected from the group consisting of formic acid, acetic acid, propionic acid, oxalic acid, HCl and HBr, more preferably it is selected from the group consisting of formic acid, acetic acid, HCl and HBr.

In a preferred embodiment the pressure above 1.5 bar during the reaction is in the range of 1.5 to 10 bar and particularly preferred in the range of 1.5 to 5 bar.

In contrast to Becker et al. the inventive process generally allows direct preparation of *N*-monosubstituted β-keto amines and addition salts of proton acids thereof. The products obtained by the inventive process can be reduced or subsequently reacted without further conversion into other salts.

The present invention is illustrated by the following non-limiting examples.

### General Procedure for Examples 1 to 8

A mixture of methyl ketone (1 equivalent (eq)), primary alkyl amine and/or an addition salt thereof (1.1 to 1.5 eq), formaldehyde (1.4 to 1.5 eq), a solvent, optionally in the presence of a proton acid, is heated in an autoclave at a total pressure above 1.5 bar for 5 to 24 hours. Afterwards, the reaction solution is cooled to 20 °C. Optionally the reaction solvent can than be removed partly or in whole and a solvent like ethyl acetate or isopropyl alcohol can be added under vigorous stirring, if necessary to facilitate precipitation of the product. The suspension is cooled (0 to 20 °C) and filtered after precipitation (0.5 to 10 hours), optionally washed and dried to afford a slightly yellow to white powder in a yield between 50 and 75 %. The product can be recrystallized from isopropyl alcohol and/or ethyl acetate if necessary. If the stability of the free base is sufficient at ambient conditions, extracting with an organic solvent and an aqueous base affords the free base.

### General Procedure for Comparative Examples 1 to 6

A mixture of methyl ketone (1 eq), primary alkyl amine and/or an addition salt thereof (1 to 1.5 eq), formaldehyde (1.0 to 1.5 eq), optionally in the presence of a proton acid, is heated in refluxing solvent for 5 to 24 hours. Afterwards, the mixture is cooled to 20 °C. Optionally the reaction solvent can than be removed partly or in whole and a solvent like ethyl acetate or isopropyl alcohol can be added under vigorous stirring, if necessary to facilitate precipitation of the product. The suspension is cooled (0 to 20 °C) and filtered after precipitation (0.5 to 10 hours), optionally washed and dried to afford a slightly yellow to white powder in a yield between 30 and 45 %. The product can be recrystallized from isopropyl alcohol and/or ethyl acetate if necessary.

### Example 1: 3-(Methylamino)-1-(thiophen-2-yl)propan-1-one hydrochloride (I, R¹ = thiophen-2-yl, R² = methyl)

2-Acetylthiophene (25.5 g, 200 mmol); methylamine hydrochloride (14.9 g, 220 mmol, 1.1 eq); paraformaldehyde (8.2 g, 280 mmol, 1.4 eq); HCl conc. (1.0 g); ethanol (100 mL); 110 °C for 9 hours; ca. 2 to 2.5 bar; removing of ethanol (50 mL) *in vacuo;* addition of ethyl acetate (200 mL); ca. 71 % yield.
¹H-NMR δ (DMSO-d₆, 400 MHz): 9.16 (2 H, s, br), 8.07 (1 H, dd, *J* = 5.0, 1.0), 8.01 (1 H, dd, *J =* 3.8, 1.0), 7.29 (1 H, dd, *J =* 5.0, 3.8), 3.49 (2 H, t), 3.20 (2 H, t), 2.56 (3 H, s).
¹³C-NMR δ (DMSO-d₆, 100 MHz): 189.9, 142.7, 135.4, 133.8, 128.8, 43.1, 34.6, 32.4.

### Example 2: 3-(Methylamino)-1-(thiophen-2-yl)propan-1-one hydrochloride (I, R¹ = thiophen-2-yl, R² = methyl)

2-Acetylthiophene (24.9 g, 197 mmol); methylamine hydrochloride (14.8 g, 219 mmol, 1.1 eq); paraformaldehyde (8.3 g, 276 mmol, 1.4 eq); HCl conc. (1.1 g); isopropyl alcohol (100 mL); 110 °C for 8 hours; ca. 2 to 2.5 bar; addition of isopropyl alcohol (50 mL); ca. 65 % yield.

### Comparative Example 1: 3-(Methylamino)-1-(thiophen-2-yl)propan-1-one hydrochloride (I, R¹ = thiophen-2-yl, R² = methyl)

2-Acetylthiophene (7.9 g, 300 mmol); methylamine hydrochloride (30.4 g, 450 mmol, 1.5 eq); paraformaldehyde (12.6 g, 420 mmol, 1.4 eq); HCl conc. (1.5 g); isopropyl alcohol (200 mL); heating under reflux (82 °C) for 8 hours; addition of ethyl acetate (200 mL); ca. 43 % yield.

### Example 3: 3-(Ethylamino)-1-(thiophen-2-yl)propan-1-onehydrochloride (I, R¹ = thiophen-2-yl, R² = ethyl)

2-Acetylthiophene (6.3 g, 50 mmol); ethylamine hydrochloride (6.1 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (25 mL) *in vacuo;* addition of ethyl acetate (50 mL); ca. 73 % yield.
¹H-NMR δ (DMSO-d₆, 400 MHz): 9.3 (2 H, s, br), 8.08 (1 H, dd), 8.00 (1 H, dd), 7.28 (1 H, dd), 3.51 (2 H, t), 3.20 (2 H, t), 2.96 (2 H, q), 1.23 (3 H, t).

### Comparative Example 2: 3-(Ethylamino)-1-(thiophen-2-yl)propan-1-onehydrochloride (I, R¹ = thiophen-2-yl, R² = ethyl)

2-Acetylthiophene (12.6 g, 100 mmol); ethylamine hydrochloride (12.2 g, 150 mmol, 1.5 eq); paraformaldehyde (4.1 g, 140 mmol, 1.4 eq); HCl conc. (0.5 g); ethanol (70 mL); heating under reflux (78 °C) for 6 hours; removing of ethanol (25 mL) *in vacuo;* addition of ethyl acetate (70 mL); ca. 31 % yield.

### Example 4: 3-(Isobutylamino)-1-(thiophen-2-yl)propan-1-onehydrochloride (I, R¹ = thiophen-2-yl, R² = isobutyl)

2-Acetylthiophene (6.3 g, 50 mmol); isobutylamine hydrochloride (8.3 g, 75 mmol, 1.5 eq); paraformaldehyde (2.1 g, 75 mmol, 1.5 eq); HCl conc. (0.3 g); ethanol (35 mL); 110 °C for 9 hours; ca. 2 to 2.5 bar; removing of ethanol (35 mL) *in vacuo;* addition of ethyl acetate (50 mL); ca. 56 % yield.
¹H-NMR δ (DMSO-d₆, 400 MHz): 9.0 (2 H, s, br), 8.08 (1 H, dd), 7.99 (1 H, dd), 7.29 (1 H, dd), 3.55 (2 H, t), 3.22 (2 H, t), 2.78 (2 H, d), 2.03 (1 H, m), 0.96 (6 H, d).

### Comparative Example 3: 3-(Isobutylamino)-1-(thiophen-2-yl)propan-1-one hydrochloride (I, R¹ = thiophen-2-yl, R² = isobutyl)

2-Acetylthiophene (12.6 g, 100 mmol); isobutylamine hydrochloride (16.5 g, 150 mmol, 1.5 eq); paraformaldehyde (4.1 g, 140 mmol, 1.4 eq); HCl conc. (0.5 g); butanol (70 mL); heating under reflux (108 °C) for 7 hours; addition of ethyl acetate (100 mL); ca. 40 % yield.

### Example 5: 3-(tert-Butylamino)-1-(thiophen-2-yl)propan-1-one hydrochloride (I, R¹ = thiophen-2-yl, R² = tert-butyl)

2-Acetylthiophene (6.3 g, 50 mmol); tert-butylamine hydrochloride (8.3 g, 75 mmol, 1.5 eq); paraformaldehyde (2.1 g, 75 mmol, 1.5 eq); HCl conc. (0.3 g); butanol (35 mL); 117 °C for 9 hours; ca. 2 to 2.5 bar; addition of ethyl acetate (50 mL); ca. 52 % yield.
¹H-NMR δ (DMSO-d₆, 400 MHz): 9.2 (2 H, s, br), 8.08 (1 H, dd), 7.98 (1 H, dd), 7.30 (1 H, dd), 3.54 (2 H, t), 3.19 (2 H, t), 1.34 (9 H, s).

### Comparative Example 4: 3-(tert-Butylamino)-1-(thiophen-2-yl)propan-1-onehydrochloride (I, R¹ = thiophen-2-yl, R² = tert-butyl)

2-Acetylthiophene (12.6 g, 100 mmol); *tert*-butylamine hydrochloride (16.5 g, 150 mmol, 1.5 eq); paraformaldehyde (4.1 g, 140 mmol, 1.4 eq); HCl conc. (0.5 g); butanol (70 mL); heating under reflux (108 °C) for 18 hours; addition of ethyl acetate (100 mL); ca. 37 % yield.

### Example 6: 3-(Methylamino)-1-(furan-2-yl)propan-1-one hydrochloride (I, R¹ = furan-2-yl, R² = methyl)

2-Acetylfuran (7.5 g, 68 mmol); methylamine hydrochloride (6.9 g, 102 mmol, 1.5 eq); paraformaldehyde (3.1 g, 102 mmol, 1.5 eq); HCl conc. (1.15 g); ethanol (35 mL); 110 °C for 8 hours; ca. 2 to 2.5 bar; removing of ethanol (30 mL) *in vacuo;* addition of ethyl acetate (50 mL); ca. 64 % yield.
¹H-NMR δ (DMSO-d₆, 400 MHz): 9.0 (2 H, s, br), 8.05 (1 H, m), 7.53 (1 H, m), 6.77 (1 H, m), 3.34 (2 H, t), 3.2 (2 H, m), 2.57 (3 H, s, br).

### Comparative Example 5: 3-(Methylamino)-1-(furan-2-yl)propan-1-one hydrochloride (I, R¹= furan-2-yl, R² = methyl)

2-Acetylfuran (11.0 g, 100 mmol); methylamine hydrochloride (10.1 g, 150 mmol, 1.5 eq); paraformaldehyde (4.1 g, 140 mmol, 1.4 eq); HCl conc. (0.5 g); butanol (70 mL); heating under reflux (108 °C) for 7 hours; addition of ethyl acetate (100 mL); ca. 44 % yield.

### Example 7: 3-(Methylamino)-1-phenylpropan-1-one hydrochloride (I, R¹ = phenyl, R² = methyl)

2-Acetophenone (21.0 g, 175 mmol); methylamine hydrochloride (17.5 g, 263 mmol, 1.5 eq); paraformaldehyde (7.9 g, 263 mmol, 1.5 eq); HCl conc. (1.1 g); ethanol (130 mL); 115 °C for 24 hours; ca. 2 to 2.5 bar; addition of ethyl acetate (170 mL); ca. 52 % yield.
¹H-NMR δ (DMSO-d₆, 400 MHz): 9.2 (2 H, s, br), 8.0 (2 H, m), 7.7 (1 H, m), 7.6 (2 H, m), 3.55 (2 H, t), 3.21 (2 H, t), 2.59 (3 H, s).

### Example 8: 3-(Methylamino)-1-(2-naphthyl)propan-1-one hydrochloride (I, R¹ = 2-naphthyl, R² = methyl)

2-Acetonaphtone (8.5 g, 50 mmol); methylamine hydrochloride (5.1 g, 75 mmol, 1.5 eq); paraformaldehyde (2.1 g, 75 mmol, 1.5 eq); HCl conc. (0.3 g); ethanol (35 mL); 117 °C for 14 hours; ca. 2 to 2.5 bar; removing of ethanol (35 mL) *in vacuo;* addition of ethyl acetate (50 mL); ca. 60 % yield.
¹H-NMR δ (DMSO-d₆, 400 MHz): 9.3 (2 H, s, br), 8.74 (1 H, s), 8.17 (1 H, d), 8.0 (3 H, m), 7.7 (2 H, m), 3.70 (2 H, t), 3.28 (2 H, m), 2.60 (3 H, s).

### Comparative Example 6: 3-(Methylamino)-1-(2-naphthyl)propan-1-one hydrochloride (I, R¹ = 2-naphthyl, R² = methyl)

2-Acetonaphtone (17.0 g, 100 mmol); methylamine hydrochloride (10.1 g, 150 mmol, 1.5 eq); paraformaldehyde (4.1 g, 140 mmol, 1.4 eq); HCl conc. (0.5 g); ethanol (70 mL); heating under reflux (78 °C) for 5 hours; removing of ethanol (30 mL) *in vacuo;* addition of ethyl acetate (100 mL); ca. 42 % yield.

## Claims

1. A process for the preparation of a compound of formula 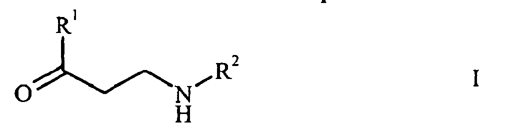 and/or an addition salt of a proton acid, wherein R¹ and R² independently represent alkyl, cycloalkyl, aryl or aralkyl, each being optionally further substituted with alkyl, alkoxy and/or halogen, which process comprises reacting
(i) a methyl ketone of formula 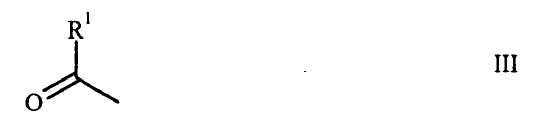 wherein R¹ is as defined above, and
(ii) a compound of formula
H₂N-R² IV
and/or an addition salt of a proton acid, wherein R² is as defined above, and
(iii) formaldehyde or a source of formaldehyde selected from the group consisting of formaldehyde in aqueous solution, 1,3,5-trioxane, paraformaldehyde and mixtures thereof, in the presence of
a solvent selected from the group consisting of water, aliphatic alcohols, cycloaliphatic alcohols and mixtures thereof, and
optionally a proton acid
to afford a β-keto amines of formula 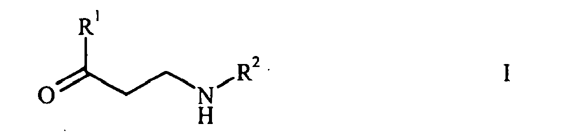 and/or an addition salt of a proton acid, wherein R¹ and R² are as defined above, and wherein the reaction is carried out at a pressure above 1.5 bar.

2. The process of claim 1 wherein R¹ is selected from the group consisting of linear or branched C₁₋₈ alkyl, C₃₋₈ cycloalkyl, phenyl, naphthyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅.

3. The process of claim 1 or 2 wherein R² is selected from the group consisting of linear or branched C₁₋₈ alkyl, C₃₋₈ cycloalkyl, phenyl, naphthyl, furanyl, benzofuranyl, thienyl, benzo[b]thienyl and aralkyl, wherein the alkyl moiety of the aralkyl residue is linear C₁₋₄ alkyl, and the aryl moiety is selected from the group consisting of phenyl, naphthyl, furanyl, benzofuranyl, thienyl and benzo[b]thienyl,
each aryl or aralkyl being optionally substituted with halogen, linear or branched C₁₋₄ alkyl, linear or branched C₁₋₄ alkoxy, C₃₋₆ cycloalkyl, CF₃, C₂F₅, OCF₃ or OC₂F₅.

4. The process of any of claims 1 to 3, wherein the compound of formula IV is present in an amount at least equimolar to that of the compound of formula III.

5. The process of any of claims 1 to 4, wherein the proton acid is a carboxylic or an inorganic acid, preferably the acid is selected from the group consisting of formic acid, acetic acid, propionic acid, oxalic acid, malonic acid, benzoic acid, HF, HCl, HBr, HI, H₂SO₄, H₃PO₄, mono alkali malonate, alkali hydrogensulfates, alkali hydrogenphosphates and alkali hydrogencarbonates.

6. The process of any of claims 4 to 5, wherein aliphatic and cycloaliphatic alcohols are selected from the group consisting of linear or branched aliphatic C₁₋₁₂ alcohols, cycloaliphatic C₅₋₈ alcohols, di- triethylene glycols and mono C₁₋₄ alkyl or acetyl derivatives thereof, each of said alcohols containing 1 to 3 hydroxy groups.

7. The process of claim 6, wherein the alcohol is selected from the group consisting of methanol, ethanol, propanol, isopropyl alcohol, butanol, isobutanol, tert-butanol, 1-pentanol, 2-pentanol, 3-pentanol, 1-hexanol, 2-hexanol, cyclopentanol, cyclohexanol, 1,2-ethanediol, 1,2-propanediol, 1,2-butanediol, 2,3-butanediol, 1,4-butanediol, 1,2,3-propanetriol, 1,2,6-hexanetriol, diethylene glycol; diethylene glycol monomethyl ether, diethylene glycol monoethyl ether, diethylene glycol monobutyl ether, diethylene glycol monoacetate, triethylene glycol, triethylene glycol monomethyl ether, triethylene glycol monoethyl ether, triethylene glycol monobutyl ether and triethylene glycol monoacetate.

8. The process of any of claims 1 to 7, wherein the pressure during the reaction is in the range of 1.5 to 10 bar.

## Patentansprüche

1. Verfahren zur Herstellung einer Verbindung der Formel 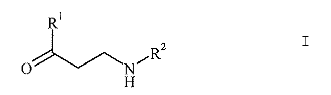 und/oder eines Protonensäureadditionssalzes, worin R¹ und R² unabhängig voneinander für Alkyl, Cycloalkyl, Aryl oder Arylakyl stehen, wobei jedes Aryl oder Aralkyl gegebenenfalls ferner durch Alkyl, Alkoxy und/oder Halogen substituiert ist, bei dem man eine Mischung, enthaltend
(i) ein Methylketon der Formel 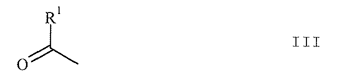 worin R¹ die oben angegebene Bedeutung besitzt, und
(ii) eine Verbindung der Formel
H₂N-R² IV
und/oder ein Protonensäureadditionssalz, worin R² die oben angegebene Bedeutung besitzt, und
(iii) Formaldehyd oder eine aus der Gruppe bestehend aus Formaldehyd in wäßriger Lösung, 1,3,5-Trioxan, Paraformaldehyd und Mischungen davon ausgewählte Formaldehyd-Quelle, in Gegenwart eines Lösungsmittels aus der Gruppe bestehend aus Wasser, aliphatischen Alkoholen, cycloaliphatischen Alkoholen und Mischungen davon und gegebenenfalls einer Protonensäure zu einem β-Ketoamin der Formel 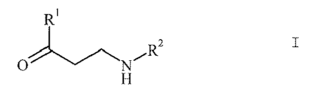 und/oder einem Protonensäureadditionssalz umsetzt und wobei man die Reaktion bei einem Druck über 1,5 bar durchführt.

2. Verfahren nach Anspruch 1, bei dem R¹ aus der Gruppe bestehend aus linearem oder verzweigtem C₁₋₈-Alkyl, C₃₋₈-Cycloalkyl, Phenyl, Naphthyl, Furanyl, Benzofuranyl, Thienyl, Benzo[b]thienyl und Aralkyl stammt, wobei der Alkylteil des Aralkylrests lineares C₁₋₄-Alkyl ist und der Arylteil aus der Gruppe bestehend aus Phenyl, Naphthyl, Furanyl, Benzofuranyl, Thienyl und Benzo[b]thienyl stammt,
wobei jedes Aryl oder Aralkyl gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist.

3. Verfahren nach Anspruch 1 oder 2, bei dem R² aus der Gruppe bestehend aus linearem oder verzweigtem C₁₋₈-Alkyl, C₃₋₈-Cycloalkyl, Phenyl, Naphthyl, Furanyl, Benzofuranyl, Thienyl, Benzo[b]thienyl und Aralkyl stammt, wobei der Alkylteil des Aralkylrests lineares C₁₋₄-Alkyl ist und der Arylteil aus der Gruppe bestehend aus Phenyl, Naphthyl, Furanyl, Benzofuranyl, Thienyl und Benzo[b]thienyl stammt,
wobei jedes Aryl oder Aralkyl gegebenenfalls durch Halogen, lineares oder verzweigtes C₁₋₄-Alkyl, lineares oder verzweigtes C₁₋₄-Alkoxy, C₃₋₆-Cycloalkyl, CF₃, C₂F₅, OCF₃ oder OC₂F₅ substituiert ist.

4. Verfahren nach einem der Ansprüche 1 bis 3, bei dem die Verbindung der Formel IV in einer zur Menge der Verbindung der Formel III zumindest äquimolaren Menge vorliegt.

5. Verfahren nach einem der Ansprüche 1 bis 4, bei dem es sich bei der Protonensäure um eine Carbonsäure oder eine anorganische Säure handelt, die vorzugsweise aus der Gruppe bestehend aus Ameisensäure, Essigsäure, Propionsäure, Oxalsäure, Malonsäure, Benzoesäure, HF, HCl, HBr, HI, H₂SO₄, H₃PO₄, Monoalkalimalonat, Alkalihydrogensulfaten, Alkalihydrogenphosphaten und Alkalihydrogencarbonaten stammt.

6. Verfahren nach einem der Ansprüche 1 bis 5, bei dem aliphatische und cycloaliphatische Alkohole aus der Gruppe bestehend aus linearen oder verzweigten C₁₋₁₂-Alkoholen, cycloaliphatischen C₅₋₈-Alkoholen, Di- und/oder Triethylenglykolen und Mono-C₁₋₄- oder Acetylderivaten davon stammen, wobei jeder der Alkohole 1 bis 3 Hydroxygruppen enthält.

7. Verfahren nach Anspruch 6, bei dem der Alkohol aus der Gruppe bestehend aus Methanol, Ethanol, Propanol, Isopropylalkohol, Butanol, Isobutanol, tert-Butanol, 1-Pentanol, 2-Pentanol, 3-Pentanol, 1-Hexanol, 2-Hexanol, Cyclopentanol, Cyclohexanol, 1,2-Ethandiol, 1,2-Propandiol, 1,2-Butandiol, 2,3-Butandiol, 1,4-Butandiol, 1,2,3-Propantriol, 1,2,6-Hexantriol, Diethylenglykol, Diethylenglykolmonomethylether, Diethylenglykolmonoethylether, Diethylenglykolmonobutylether, Diethylenglykolmonoacetat, Triethylenglykol, Triethylenglykolmonomethylether, Triethylenglykolmonoethylether, Triethylenglykolmonobutylether und Triethylenglykolmonoacetat stammt.

8. Verfahren nach einem der Ansprüche 1 bis 7, bei dem der Druck während der Reaktion im Bereich von 1,5 bis 10 bar liegt.

## Revendications

1. Procédé pour la préparation d'un composé de formule 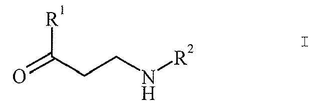 et/ou d'un sel d'addition d'un acide protonique, dans laquelle R¹ et R² représentent indépendamment un groupe alkyle, un groupe cycloalkyle, un groupe aryle ou un groupe aralkyle, chaque groupe aryle ou aralkyle étant en outre éventuellement substitué par un groupe alkyle, un groupe alcoxy et/ou un atome d'halogène, lequel procédé comprend la réaction d'un mélange comprenant :
(i) une méthylcétone de formule 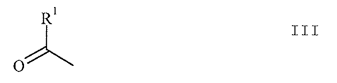 dans laquelle R¹ est tel que défini ci-dessus, et
(ii) un composé de formule
H₂N-R² IV
et/ou un sel d'addition d'un acide protonique, dans laquelle R² est tel que défini ci-dessus, et
(iii) du formaldéhyde ou une source de formaldéhyde choisie parmi le groupe constitué du formaldéhyde en solution aqueuse, du 1,3,5-trioxane, du para-formaldéhyde et de leurs mélanges, en présence :
d'un solvant choisi parmi le groupe constitué de l'eau, d'alcools aliphatiques, d'alcools cycloaliphatiques et de leurs mélanges, et
éventuellement, d'un acide protonique,
pour donner lieu à une β-cétoamine de formule 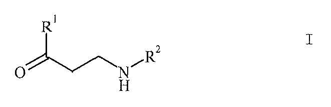 et/ou un sel d'addition d'un acide protonique, dans laquelle R¹ et R² est tel que défini ci-dessus, où la réaction est réalisée à une pression supérieure à 1,5 bar.

2. Procédé selon la revendication 1, dans lequel R¹ est choisi parmi le groupe constitué d'un groupe alkyle en C₁₋₈ linéaire ou ramifié, d'un groupe cycloalkyle en C₃₋₈, d'un groupe phényle, d'un groupe naphtyle, d'un groupe furanyle, d'un groupe benzofuranyle, d'un groupe thiényle, d'un groupe benzo[b]thiényle et d'un groupe aralkyle, où le fragment alkyle du résidu aralkyle est un groupe alkyle en C₁₋₄ linéaire, et le fragment aryle est choisi parmi le groupe constitué d'un groupe phényle, d'un groupe naphtyle, d'un groupe furanyle, d'un groupe benzofuranyle, d'un groupe thiényle et d'un groupe benzo[b]thiényle,
chaque groupe aryle ou aralkyle étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅.

3. Procédé selon la revendication 1 ou 2, dans lequel R² est choisi parmi le groupe constitué d'un groupe alkyle en C₁₋₈ linéaire ou ramifié, d'un groupe cycloalkyle en C₃₋₈, d'un groupe phényle, d'un groupe naphtyle, d'un groupe furanyle, d'un groupe benzofuranyle, d'un groupe thiényle, d'un groupe benzo[b]thiényle et d'un groupe aralkyle, où le fragment alkyle du résidu aralkyle est un groupe alkyle en C₁₋₄ linéaire, et le fragment aryle est choisi parmi le groupe constitué d'un groupe phényle, d'un groupe naphtyle, d'un groupe furanyle, d'un groupe benzofuranyle, d'un groupe thiényle et d'un groupe benzo[b]thiényle,
chaque groupe aryle ou aralkyle étant éventuellement substitué par un atome d'halogène, un groupe alkyle en C₁₋₄ linéaire ou ramifié, un groupe alcoxy en C₁₋₄ linéaire ou ramifié, un groupe cycloalkyle en C₃₋₆, un groupe CF₃, un groupe C₂F₅, un groupe OCF₃ ou un groupe OC₂F₅.

4. Procédé selon l'une quelconque des revendications 1 à 3, dans lequel le composé de formule IV est présent en une quantité au moins équimolaire par rapport du celle du composé de formule III.

5. Procédé selon l'une quelconque des revendications 1 à 4, dans lequel l'acide protonique est un acide carboxylique ou un acide inorganique, l'acide étant de préférence choisi parmi le groupe constitué de l'acide formique, de l'acide acétique, de l'acide propionique, de l'acide oxalique, de l'acide malonique, de l'acide benzoïque, du HF, du HCl, du HBr, du HI, du H₂SO₄, du H₃PO₄, d'un malonate mono-alcalin, d'hydrogénosulfates alcalins, d'hydrogénophosphates alcalins et d'hydrogénocarbonates alcalins.

6. Procédé selon l'une quelconque des revendications 1 à 5, dans lequel les alcools aliphatiques et cycloaliphatiques sont choisis parmi le groupe constitué d'alcools en C₁₋₁₂ aliphatiques linéaires ou ramifiés, d'alcools en C₅₋₈ cycloaliphatiques, de di- et/ou de tri-éthylèneglycols et de leurs dérivés mono-C₁₋₄-alkyliques ou acétyliques, chacun desdits alcools renfermant de 1 à 3 groupes hydroxy.

7. Procédé selon la revendication 6, dans lequel l'alcool est choisi parmi le groupe constitué du méthanol, de l'éthanol, du propanol, de l'alcool isopropylique, du butanol, de l'isobutanol, du tert-butanol, du 1-pentanol, du 2-pentanol, du 3-pentanol, du 1-hexanol, du 2-hexanol, du cyclopentanol, du cyclohexanol, du 1,2-éthanediol, du 1,2-propanediol, du 1,2-butanediol, du 2,3-butanediol, du 1,4-butanediol, du 1,2,3-propanetriol, du 1,2,6-hexanetriol, du diéthylèneglycol, de l'éther monométhylique de diéthylèneglycol, de l'éther monoéthylique de diéthylèneglycol, de l'éther monobutylique de diéthylèneglycol, du monoacétate de diéthylèneglycol, du triéthylèneglycol, de l'éther monométhylique de triéthylèneglycol, de l'éther monoéthylique de triéthylèneglycol, de l'éther monobutylique de triéthylèneglycol et du monoacétate de triéthylèneglycol.

8. Procédé selon l'une quelconque des revendications 1 à 7, dans lequel la pression durant la réaction est dans la plage de 1,5 à 10 bars.
